# EUROPÄISCHE PATENTANMELDUNG

(11) **EP 1 570 797 A1**
(43) Veröffentlichungstag der Anmeldung: **07.09.2005**
(21) Anmeldenummer: 05450041.8
(22) Anmeldetag: 07.03.2005
(51) Int. Cl.: A61B 17/72, A61B 17/74

(54) **Implantat, insbesondere Marknagel**

(30) Priorität: 05.03.2004 AT 3902004
(71) Anmelder: Ruprechter, Eva, 8301 Lassnitzöhne (AT); Kurcz, Helmut, 8301 Lassnitzöhne (AT)
(72) Erfinder: Ruprechter, Eva, 8301 Lassnitzöhne (AT); Kurcz, Helmut, 8301 Lassnitzöhne (AT)
(74) Vertreter: Haffner, Thomas M.

(57) **Zusammenfassung**

Bei einem Implantat, insbesondere Marknagel, mit Querbohrungen (3) für die Aufnahme von Verriegelungsschrauben sind wenigstens zwei relativ zueinander verstellbare Teile (1,7) vorgesehen, deren Querbohrungen (3,8) jeweils in eine miteinander fluchtende Lage bringbar sind, wobei wenigstens einer der beiden Teile (1,7) relativ zu dem anderen Teil (7,1) verdrehbar angeordnet ist und die beiden Teile (1,7) in einer gegen die Verriegelungsschrauben gepressten Verdrehlage relativ zueinander feststellbar sind.

## Beschreibung

Die Erfindung betrifft ein Implantat, insbesondere Marknagel, mit Querbohrungen für die Aufnahme von Verriegelungsschrauben, wobei das Implantat wenigstens zwei relativ zueinander verstellbare Teile aufweist, deren Querbohrungen jeweils in eine miteinander fluchtende Lage bringbar sind.

Als Implantate werden häufig aus Titan gefertigte Marknägel verwendet. Derartige Marknägel werden insbesondere bei transversalen Frakturen, Schräg- und Spiralfrakturen, Segmentfrakturen, Trümmerfrakturen, offene Frakturen, und insbesondere auch Frakturen mit Knochenverlust oder pathologischen Frakturen eingesetzt.

Weitere Einsatzgebiete betreffen die Korrektur von Beinlängendifferenzen, Rekonstruktionen nach Tumorresektion, Pseudoarthrosen, Metaphysen und Epiphysenfrakturen. Herkömmliche Marknägel weisen im proximalen und distalen Bereich Möglichkeiten für eine Verriegelung auf, wobei für eine vollständige Verriegelung in aller Regel jeweils zwei Verriegelungsschrauben im proximalen und im distalen Bereich angeordnet werden. Bei der Anwendung wird zunächst die passende Nagellänge bestimmt und ein Zielgerät mittels eines Adapters auf einem Nagel mit entsprechender Länge befestigt. Der Nagel wird in der Folge von Hand in das Eintrittsloch des proximalen Markraumes geführt und vorangetrieben.

Verriegelungsschrauben dienen dazu, Implantate in ihrer gewünschten Position zu halten, wobei nach der Festlegung Belastungen des Knochens als Querkräfte von den Verriegelungsschrauben aufgenommen werden müssen. Eine wirksame Festlegung und Aufnahme der Kräfte kann hiebei nur dann gewährleistet sein, wenn der Durchmesser der Schraube mit der lichten Weite der Querbohrung im Wesentlichen übereinstimmt, was insbesondere für die Gewährleistung der erforderlichen Rotationsstabilität und für die Entlastung des Knochens bei Belastung von wesentlicher Bedeutung ist. Verriegelungsschrauben, mit welchen derartige Implantate festgelegt werden, können über ihre gesamte axiale Länge ein durchgehendes Gewinde aufweisen, sodass der Gewindeaußendurchmesser bzw. Schraubenaußendurchmesser im Bereich der Querbohrung des Implantates an der Innenwand der entsprechenden Querbohrung des Implantates anliegt, um die Kräfte als Scherkräfte aufnehmen zu können.

Ebenso finden Verriegelungsschrauben Verwendung, welche einen gewindelosen Teil aufweisen, wodurch die kraftaufnehmende Fläche im beanspruchten Bereich vergrößert wird. Eine weitere wesentliche Voraussetzung für die Stabilität eines Implantats ist eine entsprechende Winkelstabilität zwischen dem Implantat und den Verriegelungsschrauben. Dadurch, dass bei herkömmlichen Verriegelungssystemen die lichte Weite der im Implantat ausgebildeten Bohrung zur Erleichterung der Einbringung der Verriegelungsschrauben ein wenig größer ist als der Querschnitt der Schraube bzw. der Querschnitt des gewindelosen Abschnitts der Schraube, ergibt sich ein Spalt zwischen der Schraube und der entsprechenden Querbohrung, welcher eine Instabilität des Implantats, beispielsweise ein Pendeln eines Marknagels im Markraum, zulässt.

Aus der US 6,221,074 B1 ist bereits ein Marknagel bekannt geworden, bei welchem die Verriegelungsschrauben winkelstabil im Marknagel fixiert sind. Es ist eine auf das Ende des Marknagels aufschiebbare Hülse vorgesehen, die mit den Bohrungen des Nagels fluchtende Durchbrechungen aufweist. Die Fixierung erfolgt derart, dass zunächst die Hülse ausgerichtet wird, sodass die Durchbrechungen der Hülse mit den Bohrungen des Nagels in Deckung gebracht werden. Danach werden die Verriegelungsschrauben in die Bohrungen bzw. Durchbrechungen eingeführt, wobei durch axiales Verschieben der Hülse relativ zum Nagel die Verriegelungsschrauben zwischen der Hülse und der die Querbohrungen begrenzenden Innenwand des Nagels festgeklemmt werden. Die axiale Verschiebeposition der Hülse relativ zum Nagel wird durch eine Feststellschraube fixiert, sodass insgesamt eine überaus hohe Winkelstabilität der Verriegelungsschrauben erreicht wird. Durch die axiale Verspannung wird insbesondere eine hohe Lagestabilität der Schrauben in axialer Richtung erzielt.

Die Erfindung zielt nun darauf ab, ein Implantat zu schaffen, bei welchem die Stabilität der Verriegelung, und insbesondere die Winkelstabilität zwischen Implantat und den in Querbohrungen des Implantates aufgenommenen Verriegelungsschrauben und auch die Rotationsstabilität erhöht werden. Im Zusammenhang mit Marknägeln soll beispielsweise ein Pendeln des Marknagels im Markraum verhindert werden. Gleichzeitig soll aber die Möglichkeit geschaffen werden, gewünschtenfalls Mikrobewegungen der Verriegelungsschrauben in axialer Richtung zuzulassen, um unter gewissen Umständen den Heilungsprozess an der Bruchstelle des Knochens zu beschleunigen.

Zur Lösung dieser Aufgabe besteht die erfindungsgemäße Ausbildung im Wesentlichen darin, dass wenigstens einer der beiden Teile relativ zu dem anderen Teil verdrehbar angeordnet ist und dass die beiden Teile in einer gegen die Verriegelungsschrauben gepressten Verdrehlage relativ zueinander feststellbar sind. Dadurch, dass wenigstens einer der beiden Teile relativ zu dem anderen Teil verdrehbar angeordnet ist, wobei beide Teile jeweils die Querbohrungen für die Aufnahme der Verriegelungsschrauben aufweisen, können die Querbohrungen der beiden Teile in eine miteinander fluchtende Lage gebracht und die Verriegelungsschrauben in den Querbohrungen beider Teile aufgenommen werden. Dadurch wird die Möglichkeit geschaffen, einen der beiden Teile nach Einbringen der Verriegelungsschrauben relativ zum anderen der beiden Teil zu verdrehen, wobei durch die Verdrehbewegung die durch die Querbohrungen hindurchgeführten Verriegelungsschrauben zwischen den beiden Teilen verspannt und festgeklemmt werden. Durch Festlegung der relativen Verdrehposition der beiden Implantatteile wird eine winkelstabile Fixierung der Verriegelungsschrauben relativ zum Implantat erreicht.

Dadurch, dass erfindungsgemäß die Verklemmung der Verriegelungsschrauben nicht durch eine axiale Verspannung, sondern durch eine durch die Verdrehung bewirke Anpressung erzielt wird, verbleibt in den Querbohrungen quer zur Längsrichtung der Verriegelungsschrauben ein Spiel, welches es den Verriegelungsschrauben bei entsprechender Belastung und der dadurch hervorgerufenen elastischen Verformung der die Klemmverbindung bildenden Teile erlaubt, sich relativ zu dem Implantat in axialer Richtung zu bewegen. Diese Mikrobewegungen werden beispielsweise auch an der Knochenbruchstelle wirksam und können den Heilungsprozess beschleunigen. Gewünschtenfalls kann das Ausmaß der zugelassenen Mikrobewegungen durch Einstellung der Press- bzw. Klemmkräfte den jeweiligen Bedürfnissen angepasst werden.

Mit Vorteil ist die Ausbildung derart getroffen, dass einer der beiden Teile um eine quer zur Längsachse der Querbohrungen verlaufende Achse relativ zu dem anderen Teil verdrehbar ist. Dadurch können besonders hohe Klemmkräfte und damit eine hohe Winkelstabilität erzielt werden.

Gemäß einer vorteilhaften Weiterbildung ist vorgesehen, dass jeder der wenigstens zwei Teile eine Mehrzahl von Querbohrungen oder Ausnehmungen aufweist, welche in eine miteinander fluchtende Lage bringbar sind. Bei dieser Ausbildung können durch Verspannen der beiden Teile gegen die Verriegelungsschrauben gleichzeitig eine Mehrzahl von Verriegelungsschrauben in winkelstabiler Weise festgelegt werden. Es ist somit lediglich erforderlich zunächst eine Mehrzahl von Verriegelungsschrauben in die Mehrzahl der miteinander fluchtenden Querbohrungen oder Ausnehmungen der beiden relativ zueinander bewegbaren Teile einzubringen, worauf durch Gegeneinanderverspannen der Teile gleichzeitig eine Mehrzahl von Verriegelungsschrauben fixiert wird.

Bevorzugt sind die relativ zueinander verdrehbaren Teile von einem zumindest über einen Teil seiner Länge mit einem Hohlraum ausgebildeten Außenteil und einem in diesem verdrehbar gelagerten Klemmbolzen gebildet. Der in dem Außenteil verdrehbar gelagerte Klemmbolzen kann hierbei entweder hohl oder voll ausgebildet sein. Zur Erhöhung der Stabilität ist es jedoch von Vorteil den Bolzen mit vollem Querschnitt auszubilden. Zur Erhöhung der Stabilität des Implantats ist es von Vorteil, wenn der Hohlraum, welcher in dem Außenteil zur Aufnahme des Bolzens ausgebildet ist, sich nicht über die gesamte Länge des Außenteils erstreckt sondern lediglich über denjenigen Bereich, in welchem die Querbohrungen für die Aufnahme der Verriegelungsschrauben vorgesehen sind. Zur Erhöhung der Stabilität ist weiters bevorzugt vorgesehen, dass der Querschnitt des Bolzens im Wesentlichen dem lichten Querschnitt des im Außenteil ausgebildeten Hohlraums entspricht.

Zur Fixierung der zur Verspannung der Verriegelungsschrauben gewählten relativen Verdrehlage der beiden Teile ist ein gesonderter Feststellmechanismus vorgesehen, welcher bevorzugt derart ausgeführt ist, dass ein gegen eine Stirnfläche des Klemmbolzens pressbarer Feststellbolzen vorgesehen ist, sodass der Klemmbolzen zwischen dem Feststellbolzen und dem Boden des Hohlraums verklemmt und in seiner Drehlage fixiert wird. Zur Aufbringung besonders hoher Presskräfte ist bevorzugt vorgesehen, dass der Feststellbolzen mit einem im Hohlraum ausgebildeten Innengewinde verschraubbar und gegen die Stirnfläche des Klemmbolzens schraubbar ist. Die Klemmwirkung kann noch weiter verbessert werden, wenn wenigstens eine der einander zugewandten Stirnflächen des Feststellbolzens und des Klemmbolzens eine reibungserhöhende Oberflächenstruktur und/oder -beschichtung aufweist. Hierbei können die einander zugewandten Stirnflächen des Feststellbolzens und des Klemmbolzens mit einer profilierten Oberfläche ausgebildet sein. Des weiteren können die einander zugewandten Stirnflächen des Feststellbolzens und des Klemmbolzens miteinander zusammenwirkende und in Drehrichtung des Feststellbolzens geneigte Keilflächen tragen.

Um die für die Klemmung erforderliche Verdrehlage des Klemmbolzens einzustellen ist bevorzugt ein Werkzeug vorgesehen, wobei der Klemmbolzen eine Aufnahme zum drehschlüssigen Eingreifen des Werkzeugs aufweist.

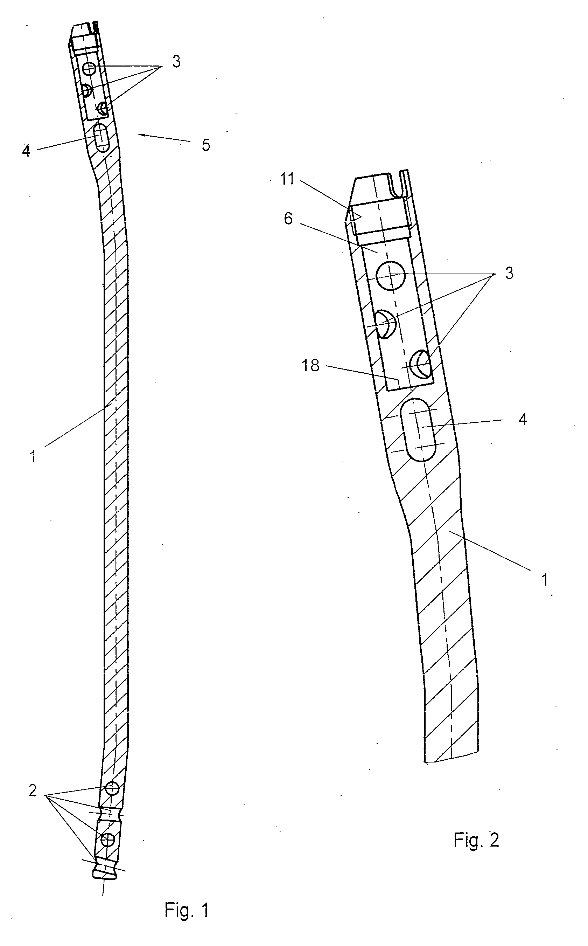

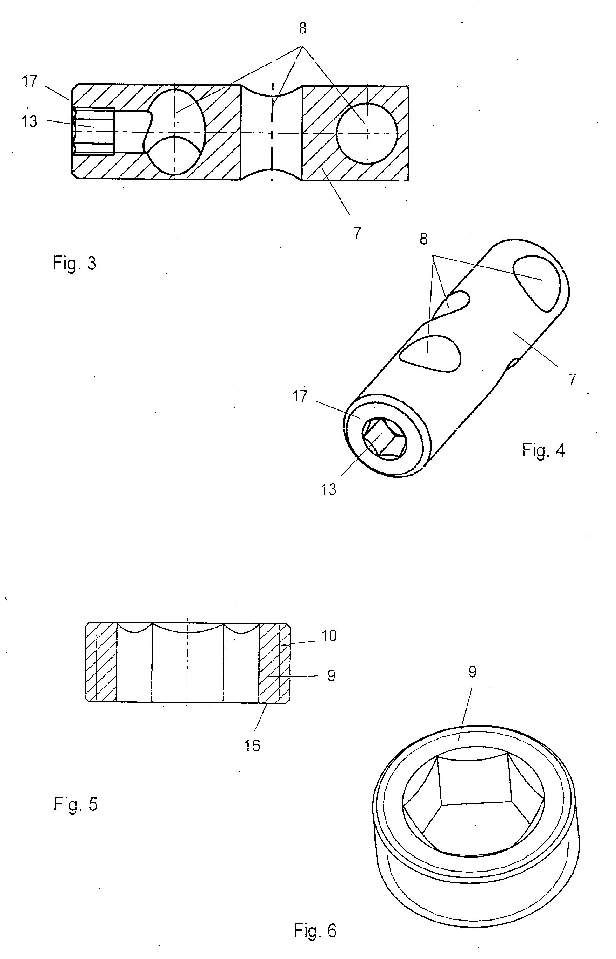

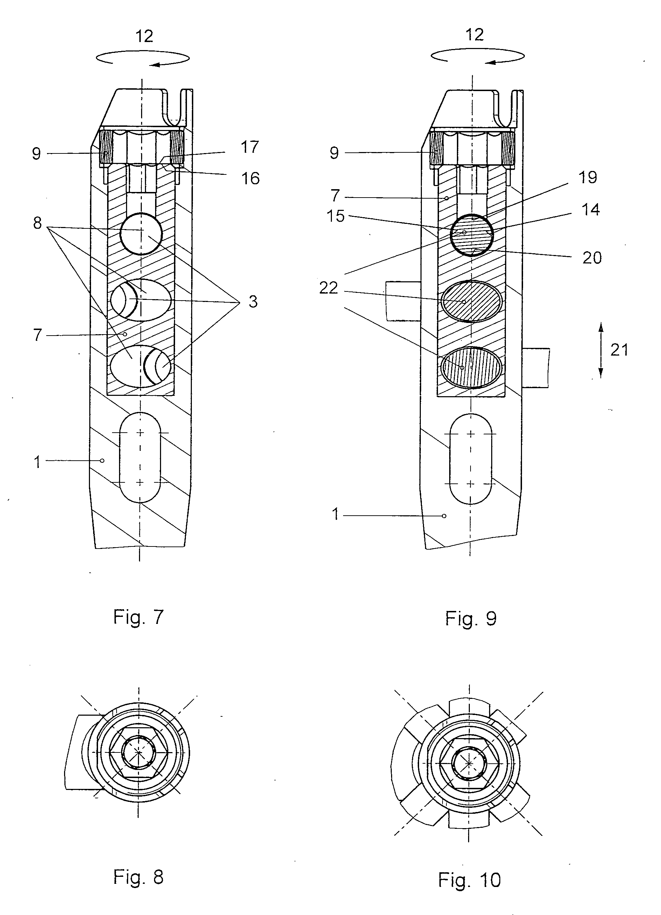

Die Erfindung wird nachfolgend anhand eines in der Zeichnung schematisch dargestellten Ausführungsbeispieles näher erläutert. In dieser zeigt Fig.1 einen Tibiamarknagel, Fig. 2 eine vergrößerte Ansicht des proximalen Endbereichs des Tibiamarknagels, Fig. 3 eine Schnittansicht eines Klemmbolzens, Fig. 4 eine perspektivische Ansicht des Klemmbolzens, Fig. 5 eine Schnittansicht eines Feststellbolzens, Fig. 6 eine perspektivische Ansicht des Feststellbolzens, Fig. 7 den proximalen Endbereich des Nagels mit eingesetztem Klemmbolzen und eingeschraubtem Feststellbolzen, Fig. 8 eine Schnittansicht des Nagels gemäß Fig. 7, Fig. 9 eine Darstellung entsprechend Fig. 7 mit eingesetzten Verriegelungsschrauben und Fig. 10 eine Schnittansicht des Nagels gemäß Fig. 9.

In Fig.1 ist ein Tibiamarknagel 1 dargestellt, weicher eine Mehrzahl von distalen Querbohrungen 2, eine Mehrzahl von proximalen Querbohrungen 3 sowie ein proximales Langloch 4 zur Aufnahme von Verriegelungsschrauben aufweist. Aus Fig. 2 ist ersichtlich, dass der Nagel 1 in seinem proximalen Endbereich 5 mit einem Hohlraum 6 ausgebildet ist. Der Hohlraum 6 dient der Aufnahme eines nachfolgend beschriebenen Klemmbolzens 7, welcher die winkelstabile Ausrichtung der in den Querbohrungen 3 aufgenommenen Verriegelungsschrauben gewährleisten soll. Der Klemmbolzen 7 ist in den Fig. 3 und 4 dargestellt und weist drei Bohrungen 8 auf, welche derart angeordnet sind, dass sie mit den Querbohrungen 3 des Nagels 1 in Deckung gebracht werden können, wenn der Klemmbolzen 7 in den Hohlraum 6 eingesetzt wird. Der Außendurchmesser des Klemmbolzens 7 entspricht im Wesentlichen dem Innendurchmesser des Hohlraums 6.

In den Fig. 5 und 6 ist ein Feststellbolzen 9 dargestellt, welcher mit einem Außengewinde 10 versehen ist und mit dem Innengewinde 11 des Hohlraums 6 verschraubt werden kann.

In der Zusammenstellungszeichnung gemäß den Fig. 7 und 9 ist ersichtlich, dass die Querbohrungen 8 des Klemmbolzens 7 mit den entsprechenden Querbohrungen 3 des Nagels 1 fluchten. Die Querbohrungen 3 und 8 sind mit einem Durchmesser ausgebildet, welcher etwas größer ist als der Außendurchmesser der in diese Querbohrungen 3 und 8 einzubringenden Verriegelungsschrauben 22, um das Einführen der Verriegelungsschrauben 22 entsprechend zu erleichtern. Das zwischen Verriegelungsschraube 22 und Querbohrung auftretende Spiel führt zu einer Unstabilität, welche erfindungsgemäß dadurch behoben wird, dass die Verriegelungsschrauben 22 zwischen den Querbohrungen 3 des Nagels 1 und den Querbohrungen 8 des Klemmbolzens 7 verklemmt werden. Dies erfolgt durch Verdrehen des Klemmbolzens 7 relativ zum Nagel 1 in Richtung des Pfeils 12, was beispielsweise mit Hilfe eines in die sechskantige Ausnehmung 13 eingreifenden Werkzeugs gelingt. Dabei werden die Verriegelungsschrauben 22 jeweils zwischen dem Bereich 14 der Innenwand der Querbohrung 8 des Klemmbolzens 7 und dem Bereich 15 der Innenwand der Querbohrung 3 des Nagels 1 verklemmt. Die für die oben genannte Verklemmung gewählte Verdrehlage des Klemmbolzens 7 wird durch den Feststellbolzen 9 gesichert, indem der Feststellbolzen 9 in Richtung des Pfeils 12 in den Hohlraum 6 eingeschraubt wird, während die gewählte Verdrehlage des Klemmbolzens 7 mit Hilfe des in die Ausnehmung 13 eingreifenden Werkzeugs beibehalten wird. Der Feststellbolzen 9 wird hierbei solange in den Hohlraum 6 eingeschraubt, bis er mit seiner unteren Stirnfläche 16 an die obere Stirnfläche 17 des Klemmbolzens 7 gepresst wird. Dadurch wird der Klemmbolzen 7 zwischen dem Feststellbolzen 9 und dem Boden 18 des Hohlraums 6 eingeklemmt, wobei die dabei auftretenden Klemmkräfte den Klemmbolzen 7 in seiner Verdrehlage fixieren.

Während die Verriegelungsschraube in den Bereichen 14 und 15 an der Innenwand der Querbohrungen 3 bzw. 8 anliegen, verbleibt in den Bereichen 19 und 20 der Querbohrungen 3 bzw. 8 ein Spiel zwischen der Verriegelungsschraube 22 und der Innenwand der Querbohrung 3 bzw. 8, sodass gewünschtenfalls in axialer Richtung entsprechend dem Doppelpfeil 21 Mikrobewegungen zugelassen werden können.

## Patentansprüche

1. Implantat, insbesondere Marknagel, mit Querbohrungen (3) für die Aufnahme von Verriegelungsschrauben, wobei das Implantat wenigstens zwei relativ zueinander verstellbare Teile (1,7) aufweist, deren Querbohrungen (3,8) jeweils in eine miteinander fluchtende Lage bringbar sind, **dadurch gekennzeichnet, dass** wenigstens einer der beiden Teile (1,7) relativ zu dem anderen Teil (7,1) verdrehbar angeordnet ist und dass die beiden Teile (1,7) in einer gegen die Verriegelungsschrauben gepressten Verdrehlage relativ zueinander feststellbar sind.

2. Implantat nach Anspruch 1, **dadurch gekennzeichnet, dass** einer der beiden Teile (1,7) um eine quer zur Längsachse der Querbohrungen (3,8) verlaufende Achse relativ zu dem anderen Teil (7,1) verdrehbar ist.

3. Implantat nach Anspruch 1 oder 2, **dadurch gekennzeichnet, dass** jeder der wenigstens zwei Teile (1,7) eine Mehrzahl von Querbohrungen (3,8) oder Ausnehmungen aufweist, welche in eine miteinander fluchtende Lage bringbar sind.

4. Implantat nach Anspruch 1,2 oder 3, **dadurch gekennzeichnet, dass** die relativ zueinander verdrehbaren Teile (1,7) von einem zumindest über einen Teil seiner Länge mit einem Hohlraum (6) ausgebildeten Außenteil (1) und einem in diesem verdrehbar gelagerten Klemmbolzen (7) gebildet sind.

5. Implantat nach einem der Ansprüche 1 bis 4, **dadurch gekennzeichnet, dass** der Querschnitt des Klemmbolzens (7) im Wesentlichen dem lichten Querschnitt des im Außenteil (1) ausgebildeten Hohlraums (6) entspricht.

6. Implantat nach einem der Ansprüche 1 bis 5, **dadurch gekennzeichnet, dass** ein gegen eine Stirnfläche (17) des Klemmbolzens (7) pressbarer Feststellbolzen (9) vorgesehen ist, sodass der Klemmbolzen (7) zwischen dem Feststellbolzen (9) und dem Boden des Hohlraums (6) verklemmt und in seiner Drehlage fixiert wird.

7. Implantat nach einem der Ansprüche 1 bis 6, **dadurch gekennzeichnet, dass** der Feststellbolzen (9) mit einem im Hohlraum (6) ausgebildeten Innengewinde (11) verschraubbar und gegen die Stirnfläche (17) des Klemmbolzens (7) schraubbar ist.

8. Implantat nach einem der Ansprüche 1 bis 7, **dadurch gekennzeichnet, dass** wenigstens eine der einander zugewandten Stirnflächen (16,17) des Feststellbolzens (9) und des Klemmbolzens (7) eine reibungserhöhende Oberflächenstruktur und/oder -beschichtung aufweist.

9. Implantat nach einem der Ansprüche 1 bis 8, **dadurch gekennzeichnet, dass** die einander zugewandten Stirnflächen (16,17) des Feststellbolzens (9) und des Klemmbolzens (7) mit einer profilierten Oberfläche ausgebildet sind.

10. Implantat nach einem der Ansprüche 1 bis 9, **dadurch gekennzeichnet, dass** die einander zugewandten Stirnflächen (16,17) des Feststellbolzens (9) und des Klemmbolzens (7) miteinander zusammenwirkende und in Drehrichtung des Feststellbolzens (9) geneigte Keilflächen tragen.

11. Implantat nach einem der Ansprüche 1 bis 10, **dadurch gekennzeichnet, dass** der Klemmbolzen (7) eine Aufnahme (13) zum drehschlüssigen Eingreifen eines Werkzeugs aufweist und dass der Feststellbolzen (9) einen im Wesentlichen ringförmigen Querschnitt aufweist.
